# EUROPEAN PATENT SPECIFICATION

(11) **EP 1 455 877 B1**
(45) Date of publication and mention of the grant of the patent: **13.08.2008**
(21) Application number: 02781282.5
(22) Date of filing: 23.10.2002
(51) Int. Cl.: A61M 16/00, A61B 5/085

(54) **Pressure-volume curve monitoring device**
Vorrichtung zur Überwachung von Druck-Volumen Kurven
Dispositif de surveillance de courbe pression-volume

(30) Priority: 30.10.2001 EP 01125888
(43) Date of publication of application: 15.09.2004
(73) Proprietor: Hamilton Medical AG, 7403 Rhäzüns (CH)
(72) Inventor: BRUNNER, Josef X., CH-7000 Chur (CH); LEI, Yuan, CH-7402 Bonaduz (CH); DURISCH, Gion,, CH-7013 Domat/Ems (CH)
(74) Representative: Dey, Michael
(86) International application number: PCT/EP2002/011869
(87) International publication number: WO 2003/037413

(56) References cited:
- WO-A-97/22377
- GB-A- 2 077 444
- US-A- 4 448 192
- US-A- 4 844 085
- US-A- 5 876 352

## Description

The present invention relates to a device for determining or/and monitoring the pressure-volume (P/V) curve of the respiratory system as well as a ventilator containing a pertinent tool for recording the pressure/volume curve.

US 5,876,352 describes a process for determining the mechanical properties of the respiratory system of a respirated patient.

The pressure/volume curve of the respiratory system is an important instrument for monitoring or diagnostic purposes. In particular, the pressure/volume curve can be used to describe the static mechanical properties of the respiratory system, especially the static compliance.

The information provided by the pressure/volume curve, in particular, enables improved ventilatory management of acute respiratory stress syndrome. Adapting ventilatory settings to individual characteristics of the patients is an extremely important aspect of better ventilatory management, particularly, a better management of patients with acute lung injury which are difficult to ventilate. Inappropriate ventilation may cause damage to the lung. Therefore, tools allowing improved determination of ventilatory settings are highly desirable. It would be especially desirable to provide means allowing to determine or monitor the pressure/volume curve also under clinical conditions in patients.

One possibility of determining the pressure/volume curve is the super syringe method. According to this, a predetermined volume is pressed into a patient's lung and the respective pressure is measured. The predetermined volume is thereby increased by predetermined increments, e.g. in 20 steps, until a final volume is reached. Subsequent to the volume increase the volume is reduced stepwise and the respective pressure is measured in this case, too. In this way a pressure/volume curve is obtained, from which the lower and upper inflection points can be determined. The problem of this method is that, due to the volume being predetermined, it cannot be excluded that a critical, lung-damaging pressure is exceeded. This is particularly applicable, if the condition of the lung is stiff. Further, since this is a static method, it has to be waited at each measurement point until no air stream is observed any longer. This means that recording the curve takes a relatively long time. In the case of a patient to be ventilated, however, the patient cannot be ventilated during the measurement period so that long measurement time is not acceptable in many cases. The problems associated with the super syringe method led to that this method is applied mainly in animal tests, however, not in clinical treatment of human patients.

Another method of determining the pressure/volume curve is the continuous flow method. Again, predetermined volumes are pressed into the lung, and the respective pressures are measured. In the case of the continuous flow method or low constant flow method, however, the volume added is not changed stepwise but slowly and continuously. While some of the problems arising in the case of the super syringe method can be overcome this way, this method still has disadvantages making it unsuitable for clinical use because of too high risks. In particular, continuous volume decrease is difficult to control. Furthermore, if the condition of the lung is stiff, very high pressures can occur, if a predetermined volume is applied, which themselves again may cause damage to the lung. Moreover, the continuous flow method, too, requires a long time to record a pressure/volume curve.

Therefore, it was an object of the present invention to provide a device enabling the recordal of a pressure/volume curve without the drawbacks existing in the case of known prior art methods. In particular, the recordal of the pressure/volume curve should be possible without any risk to the patient and possibly also in the case of patients to be ventilated.

According to the invention, this object is achieved by a device as claimed for obtaining a pressure/volume (P/V) curve of the respiratory system, wherein the applied pressure is varied and the corresponding flow or/and volumes are observed.

According to the invention predetermined pressures are applied to the respiratory system, and the flow or/and volumes corresponding to the applied pressures are measured. Since volume is a function of time and (gas or air) flow, its value can be calculated from the detected flow over time. Thus, by measuring flow and time, the corresponding volumes can be derived. The P/V curve obtained contains valuable information for e.g. a physician who in turn can use this information for diagnosis or for setting up a therapy.

The invention, thus, provides a P/V Tool in the form of a monitoring tool or monitoring feature which allows recordal of the P/V curve in a patient and is also suitable for clinical use. The device of the invention particularly allows to record the P/V curve of the respiratory system. Said curve is especially important for monitoring or diagnostic purposes. Said curve especially allows to determine the upper and lower inflection points which are parameters for adjusting the ventilator. Further, the information provided by the P/V curve offers the possibility of monitoring or/and evaluating lung conditions and lung diseases, respectively, as well as the evolution of lung diseases and then optimize ventilatory settings. Adapting ventilatory settings to individual characteristics of a patient in terms of respiratory mechanics can be an extremely important aspect of better ventilation management. In particular, potential problems and risks of ventilation such as opening-collapse phenomena and excessive lung stretch which may cause damage to the lung, can be eliminated or reduced by suitable ventilation settings. The device of the invention is a monitoring feature which allows individual determination of ventilatory settings taking into account the constraint of the respiratory system of the patient.

An essential advantage of the device of the invention is that it provides a simple and reliable technique for measuring pressure/volume curves putting only minimum strain upon a patient and do not involve any risks so that the method is suitable for clinical use. The very determination of the P/V curve in patients who are in a critical condition, as enabled by the device of the invention, however, allows to optimize ventilation parameters which is very important for these patients. The invention allows routine determination of lower and upper inflection points as well as measurement of respiratory compliance in patients with acute respiratory failure. Furthermore, repeated use of the device of the invention allows to provide physicians with data to monitor or/and diagnose lung injury severity and evolution of the lung disease.

Since according to the device of the invention it is not the volume added but the applied pressure which is varied, this device is very safe for patients. In the case of a static compliance which is other than normal and, especially stiff, the device of the invention cannot cause damages to the lung because of the predetermined pressure. Further, the device of the invention allows to record the P/V curve in a very short time. For these reasons the device is suitable for clinical use. The route of the invention of varying the pressure applied and measuring the respective volumes is a fundamental break with hitherto applied measurement devices of the prior art. This can be gathered already from the fact that in former commercially available respiratory systems a manoeuvre of varying the pressure is not provided and not possible either.

An essential aspect of the device of the invention is that the pressure is applied and predetermined and the volume found at the respective pressure is recorded as a measurement value. In a preferred embodiment, the device of the invention is carried out as follows during ventilation of a patient. First, the patient is ventilated at a predetermined minimum ventilation pressure PEEP and a predetermined inspiratory pressure. Prior to that manoeuvre prolonged expiration is carried out. During the manoeuvre the device provides a progressive pressure rise, e.g. at a predefined rate from a predetermined minimum level, e.g. atmospheric pressure (ZEEP) or another level of predetermined PEEP, to a predefined peak pressure level (Ptop). During the manoeuvre the actual pressure level and the corresponding volume can be plotted real time and continuously on a two-dimensional graph. The inflation P/V curve is then frozen and stored for later analysis. After the manoeuvre a passive expiration (deflation) to previous PEEP level is executed. Then control ventilation is resumed. By means of the data obtained the physician then can determine whether the values adjusted for ventilation are correct or whether they need to be optimized.

Since according to the device of the invention the maximum pressure applied (Pₜₒₚ) and a pressure limit not to be exceeded, respectively, can be predetermined, there is no risk of lung damage by too high pressures.

The peak pressure level Pₜₒₚ is preferably set in the range of from 20-40 cm H₂O. The ramp speed to increase pressure may be set in the range of from 1-5 cm H₂O/sec.

The above-described manoeuvre is preferably realized in such a way that the single touch of a button is sufficient for starting the program and then return to ventilation automatically. The ventilation parameters, especially PEEP and inspiratory pressure, can be adapted by the physician after evaluating the P/V curve recorded by the device of the invention. Alternatively, it is possible to provide that particular parameters are adapted automatically, with control or at least determination of a limit by the physician, however, being preferred in this case as well for safety reasons. Artificial ventilation of the patient can be resumed directly after the use of the claimed device.

The use of the P/V Tool according to the invention can be used on passive patients, i.e. in the case of patients who do not breathe spontaneously. However, it is also possible to use it in the case of active patients. Thus, the P/V curve can be recorded in the case of spontaneously breathing patients as well. The device thus can be applied in both passive patients and possibly actively breathing patients.

In a method, the pressure is varied continuously. In the case of said so-called pressure ramping the pressure is incrased from a starting pressure, e.g. from atmospheric pressure (ZEEP) or various levels of predetermined PEEP, up to a predefined peak pressure level (Ptop). The respective volume values measured are recorded thereby and be illustrated, for example, in the form of a two-dimensional graph. Alternatively, it is, of course, also possible to vary the applied pressure stepwise in predefined increments.

For recording a P/V curve according to the invention the pressure can be increased, e.g. from a predetermined minimum value to a predetermined maximum value. However, it is also possible to reduce the pressure, starting out thereby from a predefined maximum value and reducing the pressure to the desired minimum value. Depending on the information desired, it is also possible to run a cycle, wherein the pressure is increased and reduced, e.g. by first increasing from a minium value to a maximum value and then reducing it again to the minimum value, or reducing the pressure from a predefined maximum value to a predetermined minimum value and then increasing it again.

A particular advantage of the device of the invention is that recording the P/V curve requires only very short time. Particularly, it is possible to obtain the desired information within less than 30 seconds. Since ventilated patients usually are severely ill and the method of recording the P/V curve interrupts normal ventilation, it is evidently of great advantage if the manoeuvre can be performed in short time. The short duration of the use of the device, in particular, if it is automated, allows to use same also in the case of highly critical patients as well as for clinical purposes.

By the use of the device additional information can be obtained which are of interest for diagnosis and for adjusting the ventilator such as the volume at minimum ventilation pressure (V_{PEEP}).

However, the P/V curve obtained by the device of the invention mainly serves as a tool for monitoring a patient, as a tool for determining the static compliance condition of a patient and as a tool for determining PEEP and the inspiratory pressure for ventilation.

Such device can be a device which is provided only to determine the pressure/volume curve of the respiratory system. In that case the device contains means for administering a pressure as well as means for recording the respective volumes.

Also possible is a device to support respiration or/and artificial ventilation which is referred to in the following as ventilator. Besides the usual functions of ventilators this ventilator comprises an P/V Tool of the invention. Such ventilator allows to record one or more P/V curves while the patient is ventilated, with ventilation and monitoring being possible alternatingly. In particular, the ventilation function is resumed automatically.

Such ventilators particularly serve to ventilate humans, however, it is also possible to provide ventilators for animals, especially mammals.

The invention is further illustrated by the accompanying drawings and the following Examples.

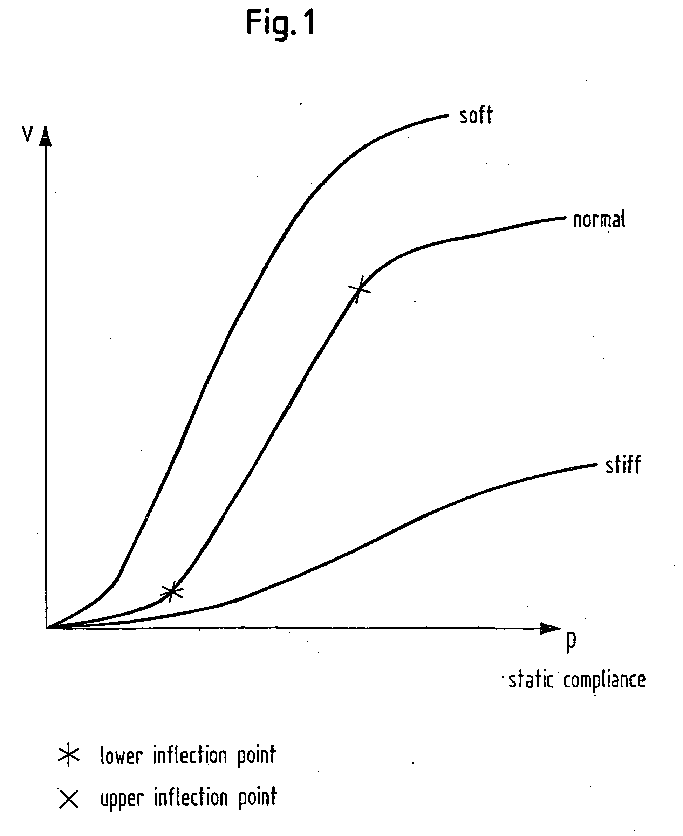

- Fig.1: is a schematic representation of a P/V curve of a normal lung, a soft lung as well as a stiff lung. Also shown are the upper and lower inflection points, between which ventilation is especially effective. It can be clearly seen from said curve that in the case of a stiff lung a predetermined volume involves the risk that very high pressures are reached, which may cause damage to the lung

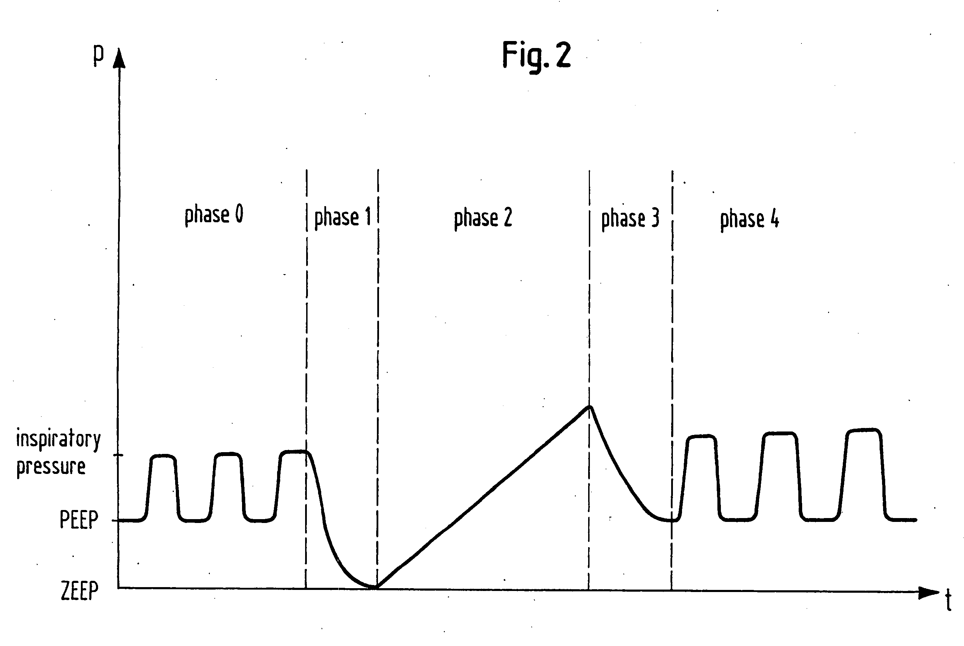

- Fig.2: shows the typical course of artificial ventilation which is interrupted by the use of the device for recording a P/V curve. It shows pressure ramping of ZEEP to a predetermined peak pressure level value Pₜₒₚ. In phase 0 artificial ventilation between values PEEP and the inspiratory pressure takes place. In phase 1 expiration to ZEEP (or another predetermined minimum value of the measurement method) is carried out. In phase 2 progressive pressure rise at a predefined rate from minimal pressure (e.g. atmospheric pressure ZEEP) to a predefined peak pressure level (Pₜₒₚ) takes place. In phase 3 passive expiration takes place (deflation) to the previous PEEP level after the manoeuvre. Then control ventilation is resumed in phase 4. The results obtained in phase 2 of the method of the invention can be used to modify and, in particular, to optimize the ventilation parameters of the patient in phase 4 or at a later stage.

### Examples

### Example 1

### Implementation of the device of the invention as a tool in a ventilator

The pressure-volume tool is a special manoeuvre to record the pressure-volume relation of the patient lungs at or close to zero flow. Basically, once activated, the device deliver a progressive pressure rise at a predefined rate from atmospheric pressure (ZEEP) to a predefined peak pressure level (Ptop). Prolonged expiration is executed before the manoeuvre, passive expiration (deflation) to the previous PEEP level after the manoeuvre. Then control ventilation is resumed. During the manoeuvre the actual pressure level and the corresponding volume are plotted real-time and continuously on a two-dimension graph. The inflation P-V curve is then frozen and stored for later analysis. Two vertical cursors which may be activated provide useful visual aid.

### Step 1: Setting

Pressing a new icon may open a special P/V Tool window for setting the parameters and starting the manoeuvre. However, the window opening does not mean start of the manoeuvre. Normal mechanical ventilation based on the control settings continues until the "Start/Stop" button is pressed.

The P/V Tool window contains the following components:
- The title "P/V Tool manoeuvre" on top
- A two-dimension graph to display on-line the measured volume at the current pressure level. The pressure is plotted as the horizontal axis and the volume as the vertical axis. The scale of the horizontal axis is fixed at the range of 0 and 40 cmH2O, whereas the vertical axis is automatically scaled.
- Two numerical values are given, one for the pressure level and another for the volume level at the point where the cursor intersects the recorded inflation curve.
- There are two setting knobs to define the manoeuvre: "P-top" (maximum pressure level at which the insufflation ends and expiration starts), and "Pramp" for pressure-ramp speed.

| Parameter | Unit | Range | Standard setting | Resolution |
|---|---|---|---|---|
| P-top | cm H2O | 20-40 | 30 | 1 |
| Ramp speed | cm H2O/second | 2..5 | 3 | 0.5 |

- The date, the time and the total manoeuvre time calculated from the set speed and the pressure range.
- A "Start/Stop" button: Pressing this button initiates the manoeuvre. If the button is pressed again during the manoeuvre, the running inflation manoeuvre is terminated immediately and passive expiration to the preset PEEP follows and control ventilation is resumed. However, the manoeuvre window remains open in this case.
- A "Close" button at the bottom of the window. If pressed, the P/V Tool window closes. Any running manoeuvre is terminated immediately and passive expiration to the preset PEEP follows and control ventilation is resumed. However, the recorded data (possibly only part) are stored.
- 2 "Cursor" buttons are to activate the cursors for analysis after the manoeuvre. These cursors are inactive during the manoeuvre.
- All above activities are controlled through the "Monitoring" knob.

### Step 2: Manoeuvre and recording

Pressing "Start/Stop" initiates the manoeuvre. The manoeuvre includes three phases.
Prolonged expiration to ensure complete lung emptying. It starts from the expiration of the last control breath. The end expiratory pressure reduces to zero (atmosphere or ZEEP) level and lasts for 5 Rce, minimally 6 seconds and maximally 15 seconds.

Immediately after the end of prolonged expiration the device performs an AutoZero and then delivers a progressive pressure increase at the preset speed, from ZEEP level to "P-top" level. The pressure-induced volume change is measured and recorded automatically. The pressure and the corresponding volume (V = O ml at ZEEP) are displayed real time as a pressure/volume curve in the graph during the manoeuvre.

After the pressure reached the "P-top" level, passive expiration follows to te previous PEEP level. The expiratory time lasts 3 x RCe which is obtained from the last control breath. The window remains open and the inflation curve is frozen for analysis.

As soon as the manoeuvre is completed, i.e. after passive expiration, normal control ventilation is resumed automatically.

At any time during the manoeuvre the operator can manually terminate the manoeuvre by pressing the "Start/Stop" button again. In this case, ventilation is resumed immediately. The part of the curve wich is already recorded, however, will still be frozen and displayed, and the window remains open.

During the manoeuvre all alarms except supply alarms (gas, power) and technical failure alarms are suppressed.

Further, the P-top setting normally overrules the Phigh limit (Pmax) setting during the manoeuvre. In this case, the actual Pmax should be automatically at P-top setting + 5 cm H2O level. However, if the level of raised Pmax is reached during the manoeuvre, the inflation manoeuvre should be aborted immediately because this may indicate the patient's activity. The device should display the message "Patient activity". The window remains open.

### Step 3: Result display and analysis

The results are displayed in the two-dimensional graph.

After the manoeuvre is completed or aborted the 2 "Cursor" buttons become active. Pressing this button, a vertical cursor appears in the graph.

Automatic calculation of UIP and LIP can be included.

The operator can move either "Cursor 1 " or "Cursor 2" by turning the M knob. The pressure and volume values corresponding to the position of the cursor along the P/V curve is shown numerically.

The window is closed either by pressing the "Close" button or automatically 60 seconds after the last movement of the M knob.

After the P-V loop window closes the PEEP level will remain at the set previous PEEP level.

It is possible to repeat the manoeuvre in the same patient, but there should be a few normal control breaths, e.g. 5, between two consecutive manoeuvres.

### 3.4.4 Review the "Last Loop"

If one manoeuvre is conducted, the recorded inflation curve should be stored together with the date and time when this manoeuvre is conducted. This curve can be replaced by a new one from the next manoeuvre.

The operator can review the stored curve at any time. When he opens the P-V curve manoeuvre window the stored curve, together with date and time, should be displayed automatically. Also, the operator may activate the cursor for analysis of the previous loop without conducting a new manoeuvre. In this case, normal ventilation is not interrupted. This window closes in the same way as after a manoeuvre.

## Claims

1. A device for obtaining a pressure/volume (PN) curve of the respiratory system, **characterized in that** it comprises
means for applying a continuously increasing or reducing predetermined pressure, and
means for measuring the corresponding flow or/and volumes.

2. A device according to claim 1, further comprising
means to record the PN curve of the respiratory system formed by the pressures applied to the respiratory system and the corresponding measured flow or/and volumes.

3. A device according to any of claim 1 or 2,
**characterized in that** said means comprises the possibility of varing the pressure from a predetermined level above, at or below atmospheric pressure to a predetermined maximum pressure.

4. A device according to claims 1-3,
wherein the predetermined pressure is applied to the respiratory system in the course of artificial ventilation.

5. A device according to any of claims 1-4,
**characterized in that** it comprises automatic resumption of the ventilation function after recordal of the pressure/volume curve.

6. The device according to any of the preceding claims,
**characterized in that** it serves as a tool for determining the static compliance condition of a patient.

7. The device according to any of the preceding claims,
**characterized in that** it serves as a tool for determining PEEP and/or the inspiratory pressure for ventilation.

## Patentansprüche

1. Vorrichtung, um eine Druck-Volumen- (P/V-) Kurve des Atmungssystems zu erhalten,
**dadurch gekennzeichnet, dass** sie
Hilfsmittel, um einen kontinuierlich ansteigenden oder abfallenden vorbestimmten Druck anzulegen und
Hilfsmittel, um den entsprechenden Fluss oder/und Volumina zu messen, umfasst.

2. Vorrichtung nach Anspruch 1, ferner umfassend
Hilfsmittel, um die P/V-Kurve des Atmungssystems, die durch die auf das Atmungssystem angelegten Drücke und den entsprechenden gemessenen Fluss oder/und Volumina gebildet wurden, zu erfassen.

3. Vorrichtung nach einem der Ansprüche 1 oder 2, **dadurch gekennzeichnet, dass** das Hilfsmittel die Möglichkeit umfasst, den Druck von einem vorbestimmten Level oberhalb oder unterhalb von Atmosphärendruck bis zu einem vorbestimmten Maximaldruck zu verändern.

4. Vorrichtung nach den Ansprüchen 1-3,
wobei der vorbestimmte Druck auf das Atmungssystem während Beatmung angelegt wird.

5. Vorrichtung nach einem der Ansprüche 1-4,
**dadurch gekennzeichnet, dass** sie die automatische Wiederaufnahme der Beatmungsfunktion nach der Erfassung der Druck-Volumen-Kurve umfasst.

6. Vorrichtung nach einem der vorhergehenden Ansprüche,
**dadurch gekennzeichnet, dass** sie als ein Hilfsmittel dient, um den Zustand der statischen Compliance eines Patienten zu bestimmen.

7. Vorrichtung nach einem der vorhergehenden Ansprüche,
**dadurch gekennzeichnet, dass** sie als ein Hilfsmittel dient, um PEEP und/oder den Einatmungsdruck für die Beatmung zu bestimmen.

## Revendications

1. Dispositif pour l'obtention d'une courbe pression/volume (P/V) du système respiratoire, **caractérisé en ce qu'**il comprend :
des moyens pour appliquer une pression prédéterminée augmentant ou diminuant de façon continue, et
des moyens pour mesurer le débit ou/et les volumes correspondants.

2. Dispositif selon la revendication 1, comprenant de plus :
des moyens pour enregistrer la courbe P/V du système respiratoire formée par les pressions appliquées au système respiratoire et les volumes ou/et débit correspondants mesurés.

3. Dispositif selon soit la revendication 1 soit la revendication 2,
**caractérisé en ce que** lesdits moyens comprennent la possibilité de faire varier la pression à partir d'un niveau prédéterminé situé au-dessus, à, ou au-dessous de la pression atmosphérique jusqu'à une pression maximale prédéterminée.

4. Dispositif selon les revendications 1-3,
**caractérisé en ce que** la pression prédéterminée est appliquée au système respiratoire au cours de la ventilation artificielle.

5. Dispositif selon les revendications 1-4,
**caractérisé en ce qu'**il comprend la reprise automatique de la fonction de ventilation après l'enregistrement de la courbe pression/volume.

6. Dispositif selon l'une quelconque des revendications 1-4,
**caractérisé en ce qu'**il sert d'outil pour déterminer l'état de compliance statique d'un patient.

7. Dispositif selon l'une quelconque des revendications précédentes,
**caractérisé en ce qu'**il sert d'outil pour déterminer la PEEP et/ou la pression inspiratoire pour la ventilation.
